# EUROPEAN PATENT APPLICATION

(11) **EP 2 062 605 A1**
(43) Date of publication of application: **27.05.2009**
(21) Application number: 08425733.6
(22) Date of filing: 18.11.2008
(51) Int. Cl.: A61M 5/00, A61M 5/178, F04B 51/00, G01F 25/00

(54) **Calibrating device for dosing machines of radioactive substances**

(30) Priority: 23.11.2007 IT BO20070777
(71) Applicant: Tema Sinergie S.r.l., 48018 Faenza RA (IT)
(72) Inventor: Berti, Mauro, 48018 Faenza (RA) (IT)
(74) Representative: Sarpi, Maurizio

(57) **Abstract**

Calibrating device for dosing machines of a radioactive fluid including a peristaltic pump (5) and a calibrated tube (24) interposed in a fluid path between a source vial (2) and a point of delivery (7), and at least one sensor (12, 13) to associates a volume of fluid transferred to a number of revolutions that pump (5).

## Description

### Field of the invention

The present invention relates to a dosing machine of radioactive substances, particularly liquids for medical or hospital use.

### Background art

Dosing machines are known with a peristaltic pump, which is passed through by a silicone tube, and which aims to transfer a determined amount of liquid that is taken from a main vial and then filled into containers, syringes or vials containing a measured dose.

The known machines are generally using peristaltic pumps operating by compression of the tube containing the liquid to be transferred, and which are preferred to other systems because they are capable of transferring the liquid without the latter being in contact with the components of pump.

In this type of pumps, the use of silicone tube is made necessary by the flexibility and elasticity of such material that makes it possible peristalsis (gradual compression of the tube) and then to transfer fluid.

However, when the silicone tubes are manufactured they present a tolerance error on the internal diameter measure that does not go below 10-15% in the best classes of accuracy, which diameter error becomes an error of the liquid volume which is transferred during the functioning of the machine.

Therefore, to achieve greater accuracy, it would be required a manual calibration procedure like the following:
- dosing with the pump of a series of volumes of liquid;
- checking with a precision balance of the volumes obtained.

Thus obtaining a coefficient of calibration that can be accepted as valid for that assembly pump/tubes.

This calibrating procedure is obviously necessary every time when a new silicone tube is applied to the peristaltic pump:
In the case of transfer of radioactive liquid, once a batch or lot of production is completed, it may be necessary, for reasons of protection against radiation, to remove the silicone tube (and the remaining tubing which makes the hydraulic circuit);
   In the particular case of injectable liquids for human use, the hydraulic circuit that comes into contact with the dosed liquid (and particularly the silicone tube), for obvious reasons of sterility must be replaced at every batch, or single supply, which usually means one or more times a day.
A manual calibration procedure of the type now described would therefore be too expensive in terms of time, as well as it would lead to inevitable human errors.

### Summary of the invention

A first aim of this invention is to overcome the limits of accuracy of the known calibration systems in the dosage of radioactive material.

The technical task and the specified aims are substantially achieved by a calibration device comprising the technical features according to one or more of the enclosed claims.

### List of drawings

Additional features and advantages of this invention will appear more apparent from the description given as an example, and therefore not limited to, of a preferred but not exclusive embodiment of the invention, as shown in the enclose drawings in which:
- Figure 1 shows schematically a dosing machine of radioactive substances provided with a calibration device according to the invention;
- Figure 2 schematically shows a diagram of control of the machine.

### Detailed description

With reference to the enclosed drawings, it is described a machine 1 for the fractioning of doses of a radioactive liquid contained in a main vial 2 and intended to be supplied to a delivery point 7 to be connected to vials or syringes 8 containing the fraction or dose of liquid.

The machine 1 includes a peristaltic pump 5 driven by a motor with encoder and a set of tubes 18, 19, 20, which connect the pump 5 respectively to vial 2, to a bottle of saline 3 and to an air filter 4.

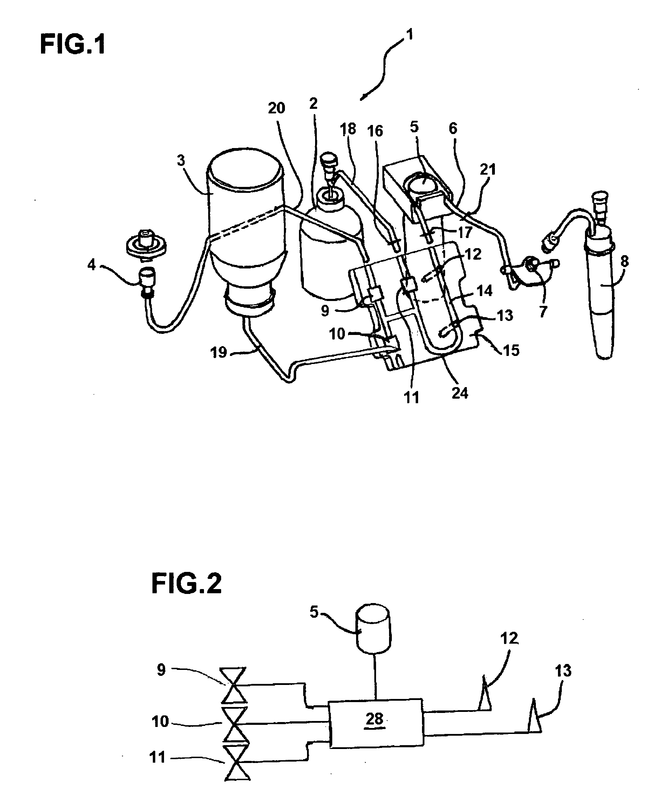

The tubes 18, 19, 20 are also interrupted by respective valves 11, 9, 10 which are controlled, as the pump 5, by an electronic control unit 28, shown in Figure 2. The unit 28 determines the opening/closing steps of the valves and the activity of pump 5 in order to transfer the desired amount of liquid, air or saline solution to the point of delivery 7.

To effect the transfer of fluid, the pump 5 operates by compression of a section of a silicon tube 6 which is delimited by two points 17 and 21 respectively upstream and downstream of the pump 5.

According to the invention, in line with the tube 6 is a section of a calibrated tube 24, preferably a single use tube, along which two sensors 12, 13 sensitive to the passage of fluid are active, so defining a measuring section 14 of tube 24.

In the example of Figure 1, the tube 24 is defined by two points 16, 17 placed upstream and downstream of the measuring section 14.

In this description, "calibrated tube" means a tube with a deviation of the actual size of internal diameter, compared with a nominal internal diameter, which is lower of the deviation of the best class of extrusion of silicone tubes: that is, with current extrusion technology, a deviation less than 10-15%.

The tube 14 is preferably attached in a removable way to a plate 15, removable in turn, and sensors 12, 13 and valves 9, 10, 11 described above are mounted on the machine body.

In the functioning, the two sensors 12, 13 delimit a volume of the calibrated tube 14 and the calibration cycle controlled by the unit 28 provides to operate the pump 5 for circulating fluid (preferably saline) to measure the revolutions of the peristaltic pump detected by the encoder and which are necessary to displace the fluid from sensor to sensor 12 to 13 i.e. to fill the volume of the calibrated section of pipe 14 between the two sensors that "see" the passage of fluid and communicate that event to drive 28

Thus, after this cycle of calibration, there will be a correspondence between the revolutions of the encoder, stored by the unit 28, and the liquid volume actually passed through the calibrated pipe, which is measured according to the calibrated dimensions of the tube and to the detection of passage the fluid by sensors 12, 13.

This correspondence allows a fast and accurate calibration of the machine in that configuration.

Advantageously, with the device of the invention the assembly peristaltic pump 5 / silicone tube 6 is calibrated again at every replacement of set of tubes (particularly of silicone tube 6), so reducing the error to less than + / - 4%, resulting from the maximum error of measurement on the diameter of the calibrated tube 14.

The invention thus conceived is susceptible of clear industrial application, may also be the subject of numerous modifications and variants all falling under the inventive concept, and all details can be replaced, moreover, by other technically equivalent.

## Claims

1. Calibrating device for dosing machines of a radioactive fluid taken from a source vial (2) and supplied to the a delivery point (7), the machine including a peristaltic pump (5) and a control unit (28), **characterized by** the fact that it comprises a tube (24) having calibrated dimensions and interposed in a fluid path between said source vial (2) and the point of delivery (7), and at least one sensor (12, 13) sensitive to the passage of a fluid through tube ( 24) for measuring a volume of fluid passing inside a section of measure (14) of the tube (24), the sensor being connected to said unit (28) in order to associate said measured volume to a number of revolutions of that pump (5).

2. Device according to claim 1, in which the calibrated tube (24) includes a section (14) extended between two flow sensors (12, 13).

3. Device according one of the previous claims, in which the peristaltic pump (5) is driven by an engine with electronically controlled encoder interfaced to said unit (28).

4. Device according to one of the previous claims, comprising a disposable tubing assembly comprising a plurality of tubes (18, 19, 20) that connect the pump (5) respectively to a vial (2), to a vial of saline (3) and to an air filter (4), and further comprising a disposable silicone tube (6) at the pump (5).

5. Device according to one of the previous claims, comprising valves (11, 9, 10) electronically controlled and arranged along said tubes (18, 19, 20) to determine the steps transfer of liquid, air or saline solution to the point of delivery (7).

6. Device according to one of the previous claims, in which the calibrated tube (24) is a disposable tube.

7. Device according to one of the previous claims, in which said disposable tubes assembly (18, 19, 20) and said calibrated tube (24) are removably attached to a plate (15)

8. Device according to claim 7, in which the plate (15) is a disposable plate.

9. Device according to one of the previous claims, in which sensors (12, 13) comprise fiber-optic sensors placed not in contact with the liquid in order to detect through the wall of the tube (24) the passage of an interface front air/liquid.

10. Device according to one of the previous claims, in which the calibrated tube (24) is a polyurethane tube with a maximum deviation on the inner diameter measure less than +/- 4%, preferably less than +/- 2%.

11. Fractioning machine of doses of radioactive substances, including a device according one or more of the previous claims.
